Europäisches Patentamt

European Patent Office

Office européen des brevets

(19)

(11) Numéro de publication : **0 173 390**
**B1**

(12)

# FASCICULE DE BREVET EUROPÉEN

(45) Date de publication du fascicule du brevet :
**01.02.89**

(21) Numéro de dépôt : **85201299.6**

(22) Date de dépôt : **09.08.85**

(51) Int. Cl.⁴ : **C 07 B 39/00**, C 07 C 25/02,
C 07 C 19/06, B 01 J 31/02

(54) Procédé pour effectuer des réactions de chloration substitutive de composés organiques au moyen de chlore moléculaire en présence d'un produit chloré servant d'initiateur radicalaire et l'utilisation des initiateurs radicalaires utilisés dans un tel procédé.

(30) Priorité : **20.08.84 FR 8413051**

(43) Date de publication de la demande :
**05.03.86 Bulletin 86/10**

(45) Mention de la délivrance du brevet :
**01.02.89 Bulletin 89/05**

(84) Etats contractants désignés :
**AT BE CH DE FR GB IT LI LU NL SE**

(56) Documents cités :
**EP--A-- 0 172 595
EP--A-- 0 172 596
FR--A-- 211 671
FR--A-- 2 190 783
CA 68: 10440a (1968)**

(73) Titulaire : **SOLVAY & Cie (Société Anonyme)
Rue du Prince Albert, 33
B-1050 Bruxelles (BE)**

(72) Inventeur : **Franklin, James
Rue Edouard Olivier, 28
B-1170 Bruxelles (BE)**

EP 0 173 390 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne un procédé pour effectuer des réactions de chloration substitutive de composés organiques au moyen de chlore moléculaire en présence d'un produit chloré servant d'initiateur radicalaire. Elle couvre également l'utilisation des initiateurs à utiliser dans un tel procédé.

On sait que les réactions de chloration substitutive des composés organiques s'effectuent souvent suivant un mécanisme radicalaire qui peut être initié par la formation d'atomes de chlore. Cela étant, ces réactions de chloration substitutive sont avantageusement exécutées dans des conditions susceptibles de favoriser la formation de chlore atomique, notamment par l'emploi de chlore moléculaire à des températures élevées au-delà de 250 °C et/ou en présence de catalyseurs ou d'initiateurs adéquats ou sous l'action de rayons actiniques, ou bien en absence de chlore moléculaire mais à l'intervention d'agents de chloration spécifiques tels que l'hexachloréthane (à 430-550 °C), le tétrachlorure de carbone (à environ 500 °C) ou le hexachlorcyclopentadiène (à 345 °C) (cf. W. DORREPAAL et R. LOUW - Int. J. Chem. Kinetics, 1978, 10, p. 249-275).

Les procédés connus présentent des inconvénients divers :

— la photochloration bien que permettant d'opérer à des températures basses, conduit à la formation de sous-produits goudronneux qui salissent les tubes lumineux et donnent lieu à une diminution subséquente du rayonnement d'initiation et dès lors du rendement de la réaction. En outre, ces procédés nécessitent d'opérer en réacteurs perméables aux rayonnements d'initiation, c'est-à-dire le plus généralement en verre, ce qui rend inexploitables des réactions réalisées à pressions élevées.

— les procédés thermiques engendrent une formation importante de goudrons avec conséquence des bouchages du réacteur ou des conduites ce qui empêche l'exécution en continu du procédé et engendre une corrosion élevée de l'appareillage.

La présente invention a pour but de remédier à ces inconvénients tout en permettant de valoriser un sous-produit gênant de certaines fabrications de produits chlorés organiques. En fait, l'invention préconise d'effectuer la chloration substitutive de composés organiques au moyen de chlore moléculaire en présence d'une faible quantité d'un initiateur nouveau qui permet d'opérer à des températures modérées, en obtenant malgré tout un excellent taux de chloration ; ledit initiateur consiste en un dérivé chloré de l'hexachlorubutadiène-1,3 qui constitue — comme on le sait — un sous-produit toxique et pratiquement dépourvu de débouchés, qui devait jusqu'ici être détruit par brûlage. Ainsi sont connues par le document Chemical Abstracts : 68, 1968, 104 400a la chloration de l'hexachlorobutadiène en $CCl_4$, $CCl_2 = CCl_2$ et $C_2Cl_6$ à 550 °C et la pyrolyse du décachlorobutane et de l'octachlorobutène en fragments $C_2$ ceux-ci étant considérés comme intermédiaires de la réaction.

L'invention concerne un procédé pour effectuer des réactions de chloration substitutive de composés organiques au moyen de chlore moléculaire en présence d'un produit chloré servant d'initiateur radicalaire dans lequel l'initiateur radicalaire utilisé résulte de la chloration additive de l'hexachlorobutadiène-1,3 et consiste en du décachlorobutane ou un octachlorobutène tel que l'octachlorobutène-1 ou un mélange contenant ces deux produits chlorés, étant exclus des composés organiques les composés organiques appartenant à la famille chimique des composés pyridiniques.

Des exemples non limitatifs de telles réactions de chloration substitutive comprennent la chloration du méthane, de l'éthane ou de leurs dérivés halogénés ainsi qu'en général la chloration de tous les composés hydrocarbonés acycliques ou cycliques ou de leurs dérivés halogénés. On peut notamment appliquer le procédé de l'invention aux composés aromatiques qui peuvent être chlorés substitutivement soit sur le noyau aromatique soit sur la chaîne latérale, soit sur ces deux positions selon les conditions opératoires choisies et bien entendu selon la nature du composé organique.

L'invention porte également sur l'utilisation des initiateurs de chloration substitutive de composés organiques, ces initiateurs consistant en un produit chloré ou un mélange de produits chlorés qui résulte de la chloration additive d'hexachlorobutadiène-1,3 et qui comprend principalement du décachlorobutane et/ou un octachlorobutène suivant le degré auquel la chloration additive a été poussée.

Lesdits produits chlorés obtenus par chloration additive d'hexachlorobutadiène-1,3 peuvent être préparés de manière connue en soi, par exemple par photochloration ou par chloration en phase liquide catalysée par le fer.

Ces chlorations conduisent à la formation de mélanges bruts qui contiennent en plus des produits principaux mentionnés plus haut, un peu d'hexachloréthane et éventuellement de l'hexachlorobutadiè-ne-1,3 non transformé, ainsi qu'une faible proportion d'autres composés.

Ainsi, à titre d'exemple, on donne ci-après la composition de mélanges bruts provenant de la photochloration de l'hexachlorobutadiène-1,3 en g/kg :

(Voir Tableau page suivante)

|  | Mélange A | Mélange B |
|---|---|---|
| Octachlorobutène-1 | 415 | - |
| Décachlorobutane | 303 | 812 |
| Hexachloréthane | 183 | 147 |
| Hexachlorobutadiène-1,3 | 77 | < 0,5 |
| Autres composés, non identifiés | 22 | 41 |

D'autre part un mélange brut obtenu par chloration d'hexachlorobutadiène-1,3 (1190 g de C$_4$Cl$_6$ et 652 g de Cl$_2$) en autoclave, à 125 °C, sous une pression de 9 bars, en présence d'environ 0,1 % en poids de FeCl$_3$ (catalyseur), le produit de la chloration ayant été lavé à l'acide chlorhydrique pour éliminer le FeCl$_3$, avait la composition suivante :

|  | Mélange C |
|---|---|
|  | g/kg |
| Octachlorobutène-1 | 200 |
| Décachlorobutane | 615 |
| Hexachloréthane | 128 |
| Hexachlorobutadiène-1,3 | 25 |
| Autres composés, non identifiés | 32 |

La quantité d'initiateur mis en œuvre dans le procédé de l'invention est comprise en général entre 0,01 et 10 moles % de la quantité totale des réactifs et diluants utilisés. Généralement cette quantité est de 0,01 à 5 moles % et de préférence on travaille avec 0,1 à 2 moles % d'initiateur par rapport aux réactifs mis en œuvre.

Le chlore moléculaire et le composé organique à chlorer sont généralement mis en œuvre dans des rapports molaires compris entre 0,1 et 20 moles de chlore par mole de composé organique. Ce rapport dépend notamment du nombre d'atomes d'hydrogène que l'on désire substituer. De préférence ce rapport varie entre 0,2 et 2 moles et tout particulièrement préférés sont des rapports molaires situés entre 0,3 et 10 moles de chlore par mole de composé organique.

Les initiateurs de chloration susdits conviennent pour les réactions en phase gazeuse ou en phase liquide. Ils peuvent être utilisés à la pression atmosphérique ou à une pression plus élevée. La température dépend bien entendu de la nature du composé à chlorer et des autres conditions opératoires, mais de toute manière

— à taux de chloration et temps de séjour donnés, l'emploi des initiateurs selon l'invention permet de réduire la température, ce qui conduit à une réduction de la formation de coke, de goudrons et de sous-produits indésirables

— à température et temps de séjour donnés, on peut atteindre un taux de chloration plus élevé, ce qui peut permettre une économie d'énergie par réduction de la quantité de substrat organique à recycler

— à température et taux de chloration donnés, on peut réduire le temps de séjour, ce qui revient à augmenter la productivité du réacteur.

On a également constaté qu'il peut être souhaitable dans certains cas afin de minimiser la surchauffe du mélange réactionnel d'exécuter la réaction de chloration en présence d'additifs qui agissent comme diluants mais qui sont inertes vis-à-vis des réactifs et des initiateurs intervenant dans la réaction. Comme additifs on utilise de préférence des dérivés chlorés aliphatiques tel que le tétrachlorure de carbone ou des produits inorganiques tels que le chlorure d'hydrogène ou l'azote.

De préférence on opère avec le tétrachlorure de carbone.

En général les additifs organiques halogénés sont ajoutés au milieu réactionnel à raison de 1 à 25 moles par mole de composé organique mis en œuvre.

Le procédé selon l'invention peut être réalisé dans tout appareillage ou tout réacteur permettant de réunir les conditions opératoires décrites ci-avant. De bons résultats ont été obtenus dans l'appareillage décrit aux exemples ci-après. Toutefois on peut travailler dans d'autres types d'appareillages selon le type de réaction réalisé notamment en vue d'améliorer la sélectivité ; ainsi on peut sélectionner selon le cas des réacteurs tels que des réacteurs tubulaires, des réacteurs homogènes en cascades...

Les exemples qui suivent illustrent l'invention sans toutefois la limiter.

Exemple 1

Dans cet exemple on a réalisé la chloration substitutive du benzène essentiellement en mono- et

3

dichlorobenzènes, à 300 °C, en absence d'initiateur (à titre d'élément de comparaison) et en présence d'un tel initiateur à savoir celui préparé par chloration d'hexachlorobutadiène-1,3 en présence de FeCl$_3$ et dont la composition a été donnée ci-avant (20 % en poids de C$_4$Cl$_8$ et 61,5 % en poids de C$_4$Cl$_{10}$) (Mélange C).

La chloration du benzène en phase gazeuse a été effectuée à la pression atmosphérique dans un réacteur mélangeur continu sphérique d'environ 1 dm$^3$, en pyrex, auto-agité par jets gazeux (Chem. Eng. Sci., 1973, 28, p. 129-137), les réactifs étant introduits sous forme gazeuse à l'aide d'un injecteur à quatre tuyères placé au milieu de la sphère.

Le réacteur est placé dans une enceinte à l'intérieur de laquelle l'air est chauffé électriquement et brassé à l'aide d'une turbine, afin de maintenir la température réactionnelle désirée. Un mélange de benzène et de CCl$_4$, ce dernier utilisé en tant que diluant inerte est alimenté via un évaporateur tubulaire vertical, chauffé électriquement, raccordé à l'injecteur. Le chlore gazeux est injecté dans le pied du tube évaporateur. L'initiateur, dans le cas où on l'a utilisé, est ajouté sous forme liquide, en solution concentrée dans du CCl$_4$. Les produits de chloration sortent du réacteur par une tubulure diamétralement opposée à l'introduction, puis ils sont condensés et traités par du NaOH aqueux pour neutraliser le chlore résiduaire et le HCl formé ; après décantation, la phase organique est séparée de la phase aqueuse et analysée par chromatographie en phase vapeur. Les conditions et les résultats des essais sont donnés au tableau 1 ci-après.

(Voir Tableau 1 pages suivantes)

Tableau 1

Chloration du benzène

|  |  | ESSAI DE REFERENCE, SANS INITIATEUR | ESSAI AVEC INITIATEUR (MELANGE C) |
|---|---|---|---|
| CONDITIONS |  |  |  |
| — température dans le réacteur | °C | 300 | 300 |
| — température sortie évaporateur $(C_6H_6 + CCl_4 + Cl_2)$ | °C | 150 | 151 |
| — introduction de l'initiateur |  | néant | solution à 38 % poids dans $CCl_4$ |
| — temps de séjour moyen (= volume du réacteur/ débit volumique des réactifs) | s | 10 | 10 |
| — rapports molaires des réactifs |  |  |  |
| . benzène | mol/mol | 1 | 1 |
| . $Cl_2$ | mol/mol | 2,0 | 2,0 |
| . $CCl_4$ (diluant inerte) | mol/mol | 3,0 | 3,0 |
| . initiateur (somme de ses constituants) | mol/mol | 0 | 0,06 |
| — teneur du mélange réactionnel en initiateur | % mol | 0 | 1,0 |

EP 0 173 390 B1

## Tableau 1 (Suite)

| | | ESSAI DE REFERENCE, SANS INITIATEUR | ESSAI AVEC INITIATEUR (MELANGE C) |
|---|---|---|---|
| RESULTATS | | | |
| − répartition des produits de chloration | | | |
| . benzène | % mol | 91,9 | 15,4 |
| . monochlorobenzène | % mol | 7,9 | 36,6 |
| . dichlorobenzènes | % mol | 0,2 | 37,9[*] |
| . trichlorobenzènes | % mol | 0 | 9,4 |
| . tétrachlorobenzènes | % mol | 0 | 0,7 |
| − conversion du benzène | % | 8 | 85 |
| − taux de chloration (= mol $Cl_2$ ayant réagi/ mol benzène mis en oeuvre) | mol/mol | 0,08 | 1,4 |

* Rapport ortho-/méta-/para- = 11/64/25

EP 0 173 390 B1

On voit, d'après ces résultats, que l'addition de 1,0 % mol d'initiateur selon l'invention, par rapport à la totalité des corps participant à la réaction ($C_6H_6$ + $Cl_2$ + $CCl_4$), permet :

— d'augmenter la conversion du benzène de 8 à 85 % ;

— d'augmenter le taux de chloration de 0,08 à 1,4 mol/mol.

Sans initiateur, le monochlorbenzène est le principal produit de la chloration ; en présence de l'initiateur, il se forme un mélange de mono-, di- et tri-chlorobenzènes.

Exemple 2

On a effectué des essais de chloration de chloroforme en tétrachlorure de carbone, à 220 et 250 °C, sous la pression atmosphérique, dans l'installation de l'exemple 1, en l'absence et en présence de l'initiateur selon l'invention et en présence d'hexachloréthane à titre de comparaison. Les initiateurs lorsqu'ils ont été utilisés, sont introduits sous forme d'une solution dans le $CHCl_3$. On a utilisé ici un défaut de $Cl_2$ par rapport au chloroforme, l'excès de ce dernier servant de diluant. Les conditions et les résultats des essais sont repris au tableau 2. L'initiateur utilisé est le même que celui de l'exemple 1.

(Voir Tableau 2 pages suivantes)

Tableau 2

Chloration du chloroforme

| | | (220°C) ESSAI DE REF. SANS INITIATEUR | (220°C) ESSAI AVEC INITIATEUR (MELANGE C) | (250°C) ESSAI DE REF. SANS INITIATEUR | (250°C) ESSAI AVEC INITIATEUR (MELANGE C) | (220°C) ESSAI DE REFERENCE AVEC HEXACHLOR-ETHANE |
|---|---|---|---|---|---|---|
| CONDITIONS | | | | | | |
| – température dans le réacteur | °C | 220 | 220 | 250 | 250 | 220 |
| – température sortie de l'évaporateur (CHCl$_3$ + Cl$_2$) | °C | 156 | 155 | 155 | 155 | 170 |
| – temps de séjour moyen | s | 10 | 10 | 10 | 10 | 10 |
| – rapports molaires des réactifs | | | | | | |
| . chloroforme | mol/mol | 1 | 1 | 1 | 1 | 1 |
| . Cl$_2$ | mol/mol | 0,33 | 0,34 | 0,33 | 0,33 | 0,33 |
| . initiateur (somme de ses constituants) | mol/mol | 0 | 0,025 | 0 | 0,026 | 0,024 |
| – teneur du mélange réactionnel en initiateur | % mol | 0 | 1,8 | 0 | 1,9 | 1,8 |

EP 0 173 390 B1

Tableau 2 (suite)

| | | (220°C) ESSAI DE REF. SANS INITIATEUR | (220°C) ESSAI AVEC INITIATEUR (MELANGE C) | (250°C) ESSAI DE REF. SANS INITIATEUR | (250°C) ESSAI AVEC INITIATEUR (MELANGE C) | (220°C) ESSAI DE REFERENCE AVEC HEXACHLOR-ETHANE |
|---|---|---|---|---|---|---|
| RESULTATS | | | | | | |
| – répartition des produits de chloration | | | | | | |
| . chloroforme | % mol | 99,0 | 82,3 | 97,1 | 77,1 | 99,4 |
| . tétrachlorure de carbone | % mol | 1,0 | 17,7 | 2,9 | 22,9 | 0,6 |
| – $CCl_4$ formé/$Cl_2$ mis en oeuvre | mol/mol | 0,03 | 0,52 | 0,09 | 0,69 | 0,02 |

9

A 220 °C, l'addition de 1,8 % mol d'initiateur selon l'invention permet d'augmenter le rapport CCl₄ formé/Cl₂ mis en œuvre d'environ 0,02 ou de 0,03 à 0,52 mol/mol.

On remarquera que l'addition de 1,8 % vol. d'hexachloréthane à 220 °C reste sans effet sur le rapport CCl₄ formé/Cl₂ mis en œuvre.

A 250 °C, ce même rapport passe de 0,09 mol/mol, en l'absence d'initiateur, à 0,69 mol/mol, avec 1,9 % mol de produit de chloration de l'hexachlorobutadiène-1,3.

## Revendications

1. Procédé pour effectuer des réactions de chloration substitutive de composés organiques au moyen de chlore moléculaire en présence d'un produit chloré servant d'initiateur radicalaire et résultant de la chloration additive de l'hexachlorobutadiène-1,3, caractérisé en ce que l'initiateur radicalaire utilisé consiste en du décachlorobutane ou un octachlorobutène tel que l'octachlorobutène-1 ou un mélange contenant ces deux produits, étant exclus des composés organiques les composés organiques appartenant à la famille chimique des composés pyridiniques.

2. Procédé suivant la revendication 1 caractérisé en ce qu'on effectue la chloration substitutive à une température supérieure à 200 °C.

3. Procédé suivant la revendication 1 ou 2 caractérisé en ce que la quantité d'initiateur mis en œuvre est comprise entre 0,01 et 10 moles % de la quantité totale des réactifs et diluants mis en œuvre.

4. Procédé suivant l'une des revendications 1 ou 2 caractérisé en ce qu'on effectue la chloration substitutive à une pression supérieure à la pression atmosphérique.

5. Procédé suivant l'une des revendications 1 à 4 caractérisé en ce qu'il est appliqué à la chloration substitutive du benzène pour obtenir du monochlorobenzène et des dichlorobenzènes.

6. Procédé suivant l'une des revendications 1 à 4 caractérisé en ce qu'il est appliqué à la chloration substitutive du chloroforme pour obtenir du tétrachlorure de carbone.

7. Utilisation comme initiateur d'un produit chloré ou un mélange de produits chlorés qui résulte de la chloration additive d'hexachlorobutadiène-1,3, et qui comprend principalement du décachlorobutane et/ou un octachlorobutène tel que l'octachlorobutène-1 suivant le degré auquel la chloration additive a été poussée, pour l'initiation de réactions de chloration substitutive de composés organiques selon la revendication 1.

8. Utilisation suivant la revendication 7 caractérisée en ce que l'initiateur est obtenu par photochloration d'hexachlorobutadiène-1,3.

9. Utilisation suivant la revendication 7 caractérisée en ce que l'initiateur est obtenu par chloration d'hexachlorobutadiène-1,3 en phase liquide, catalysée par le fer.

## Claims

1. Process for carrying out substitution chlorination reactions of organic compounds by means of molecular chlorine in the presence of a chlorinated product serving as a radical initiator and resulting from the addition chlorination of 1,3-hexachlorobutadiene, characterized in that the radical initiator used is decachlorobutane or an octachlorobutene such as octachloro-1-butene or a mixture containing these two products, the organic compounds belonging to the chemical group of pyridine compounds being excluded from the organic compounds.

2. Process according to Claim 1, characterized in that the substitution chlorination is carried out at a temperature above 200 °C.

3. Process according to Claim 1 or 2, characterized in that the quantity of initiator employed is between 0.01 and 10 mole % of the total quantity of the reactants and diluents employed.

4. Process according to either of Claims 1 or 2, characterized in that the substitution chlorination is carried out at a pressure above atmospheric pressure.

5. Process according to one of Claims 1 to 4, characterized in that it is applied to the substitution chlorination of benzene to obtain monochlorobenzene and dichlorobenzenes.

6. Process according to one of Claims 1 to 4, characterized in that it is applied to the substitution chlorination of chloroform to obtain carbon tetrachloride.

7. Use as initiator of a chlorinated product or a mixture of chlorinated products which results from the addition chlorination of hexachloro-1,3-butadiene, and which comprises principally decachlorobutane and/or an octachlorobutene such as octachloro-1-butene, depending on the degree to which the addition chlorination has been taken, for the initiation of substitution chlorination reactions of organic compounds according to Claim 1.

8. Use according to Claim 7, characterized in that the initiator is obtained by photochlorination of hexachloro-1,3-butadiene.

9. Use according to Claim 7, characterized in that the initiator is obtained by iron-catalysed liquid phase chlorination of hexachloro-1,3-butadiene.

**Patentansprüche**

1. Verfahren zur Ausführung von substitutiven Chlorierungsreaktionen von organischen Verbindungen unter Zuhilfenahme von molekularem Chlor in Gegenwart eines chlorierten Produkts, das als radikalartiger Initiator dient und aus der zusätzlichen Chlorierung von Hexachlorobutadien-1,3 resultiert, dadurch gekennzeichnet, daß der verwendete radikalartige Initiator aus Dekachlorobutan oder einem Octachlorobuten wie dem Octachlorobuten-1 oder einer Mischung enthaltend diese zwei Produkte besteht, wobei von den organischen Verbindungen die organischen Verbindungen, die zu der chemischen Familie der Pyridinverbindungen gehören, ausgeschlossen sind.

2. Verfahren nach Anspruch 1, dadurch gekennzeichnet, daß man die substitutive Chlorierung bei einer Temperatur über 200 °C bewirkt.

3. Verfahren nach Anspruch 1 oder 2, dadurch gekennzeichnet, daß die Menge des eingesetzten Initiators zwischen 0,01 und 10 Mol% der gesamten Menge der Reagenzien und eingesetzten Verdünnungsmittel beträgt.

4. Verfahren nach einem der Ansprüche 1 oder 2, dadurch gekennzeichnet, daß man die substitutive Chlorierung bei einem Druck über dem atmosphärischen Druck bewirkt.

5. Verfahren nach einem der Ansprüche 1 bis 4, dadurch gekennzeichnet, daß es auf die substitutive Chlorierung von Benzol angewandt wird, um Monochlorbenzol und Dichlorbenzole zu erhalten.

6. Verfahren nach einem der Ansprüche 1 bis 4, dadurch gekennzeichnet, daß es auf die substitutive Chlorierung von Chloroform angewandt wird, um Tetrachlorkohlenstoff zu erhalten.

7. Verwendung als Initiator eines chlorierten Produkts oder einer Mischung von chlorierten Produkten, das aus der zusätzlichen Chlorierung von Hexachlorobutadien-1,3 resultiert und das hauptsächlich Dekachlorobutan und/oder ein Octachlorobuten wie das Octochlorobuten-1 enthält gemäß dem Grad nach dem die zusätzliche Chlorierung betrieben worden ist, zur Initiation von Reaktionen der substitutiven Chlorierung von organischen Verbindungen nach Anspruch 1.

8. Verwendung nach Anspruch 7, dadurch gekennzeichnet, daß der Initiator durch Photochlorierung von Hexachlorobutadien-1,3 erhalten wird.

9. Verwendung nach Anspruch 7, dadurch gekennzeichnet, daß der Initiator durch Chlorierung von Hexachlorobutadien-1,3 in flüssiger Phase, katalysiert durch Eisen erhalten wird.